Europäisches Patentamt

European Patent Office

Office européen des brevets

(19)

(11) **EP 1 123 384 B1**

(12) **EUROPEAN PATENT SPECIFICATION**

(45) Date of publication and mention
of the grant of the patent:
**07.04.2004 Bulletin 2004/15**

(51) Int Cl.[7]: **C12N 5/00**

(86) International application number:
**PCT/US1999/026773**

(21) Application number: **99961649.3**

(22) Date of filing: **10.11.1999**

(87) International publication number:
**WO 2000/027998 (18.05.2000 Gazette 2000/20)**

(54) **USE OF COMPOSITIONS DEPLETED OF DENDRITIC CELLS FOR IMPROVING ALLOGENEIC HEMATOPOIETIC CELL TRANSPLANTATION**

VERWENDUNG VON DENDRITISCHEN ZELLEN-DEPLETIERTEN ZUSAMMENSETZUNGEN ZUR VERBESSERUNG DER TRANSPLANTATION VON ALLOGENEN HÄMATOPOIETISCHEN ZELLEN

UTILISATION DE COMPOSITIONS DEPLETEES EN CELLULES DENDRITIQUES POUR AMELIORER UNE TRANSPLANTATION DE CELLULE HEMATOPOIETIQUE ALLOGENIQUE

(84) Designated Contracting States:
**AT BE CH CY DE DK ES FI FR GB GR IE IT LI LU MC NL PT SE**

(30) Priority: **11.11.1998 US 189577**

(43) Date of publication of application:
**16.08.2001 Bulletin 2001/33**

(73) Proprietor: **Emory University
Atlanta, GA 30322 (US)**

(72) Inventor: **WALLER, Edmund, K.
Atlanta, GA 30306 (US)**

(74) Representative: **Land, Addick Adrianus Gosling
Arnold & Siedsma,
Advocaten en Octrooigemachtigden,
Sweelinckplein 1
2517 GK Den Haag (NL)**

(56) References cited:
**WO-A-95/09230**

• **CAGNONI P J ET AL: "MOBILIZATION AND SELECTION OF CD34-POSITIVE HEMATOPOIETIC PROGENITORS" BLOOD REVIEWS,GB,EDINBURGH, vol. 10, no. 1, March 1996 (1996-03), pages 1-7, XP000856623**

• **J. STOREK ET AL: "Allogeneic Peripheral Blood Stem Cell Transplantation May Be Associated With a High Risk of Chronic Graft-Versus-Host Disease" BLOOD, vol. 90, no. 12, 15 December 1997 (1997-12-15), pages 4705-4709, XP002136138**

• **BERNHARD ET AL: "GENERATION OF IMMUNOSTIMULATORY DENDRITIC CELLS FROM HUMAN CD34+ HEMATOPOIETIC PROGENITOR CELLS OF THE BONE MARROW AND PERIPHERAL BLOOD" CANCER RESEARCH,US,AMERICAN ASSOCIATION FOR CANCER RESEARCH, BALTIMORE, MD, vol. 55, 1 March 1995 (1995-03-01), pages 1099-1104, XP002085385 ISSN: 0008-5472**

• **J. OLWEUS ET AL: "Dendritic cell ontogeny: A human dendritic cell lineage of myeloid origin" PROCEDINGS OF THE NATIONAL ACADEMY OF SCIENCE USA, vol. 94, November 1997 (1997-11), pages 12551-12556, XP002136139 cited in the application**

• **G. GROUARD ET AL: "The Enigmatic Plasmacytoid T Cells develop into Dendritic Cells with Interleukin (IL)-3 and CD40-Ligand " JOURNAL OF EXPERIMENTAL MEDICINE, vol. 185, no. 6, 17 March 1997 (1997-03-17), pages 1101-1111, XP002136140 TOKYO, JP cited in the application**

• **D. STRUNK ET AL: "A Skin Homing Molecule Defines the Langerhans Cell Progenitor in Human Peripheral Blood" JOURNAL OF EXPERIMENTAL MEDICINE, vol. 185, no. 6, 17 March 1997 (1997-03-17), pages 1131-1136, XP002136141 TOKYO, JP cited in the application**

**Description**

**FIELD OF THE INVENTION**

The field of this invention is improved methods of transplanting allogeneic hematopoietic system reconstituting cells from donors to genetically distinct recipients and involves the removal of certain donor hematopoietic cell types, the presence of which are correlated with reduced survival of transplant recipients.

**BACKGROUND OF THE INVENTION**

Allogeneic bone marrow transplantation is the preferred treatment for a variety of malignant and genetic diseases of the blood and blood-forming cells. The widespread application of this therapy is limited by the availability of suitable bone marrow donors who are genetically related to the patient and share the same transplantation antigens on the surface of their blood cells. Only 25% of patients have a sibling who is an antigenically matched potential donor. Bone marrow transplantation can be offered to those patients who lack an appropriate sibling donor by using bone marrow from antigenically matched, genetically unrelated donors (identified through a national registry), or by using bone marrow from a genetically related sibling or parent whose transplantation antigens differ by one to three of six human leukocyte antigens from those of the patient. However, using antigenically mismatched, genetically related parent or sibling or antigenically matched, genetically unrelated donors, the likelihood of fatal graft vs. host disease (GvHD) and/or graft rejection increases from 20% for matched sibling donors to 50% in the cases of matched, unrelated donors and un-matched donors from the patient's family. Further, in cases where an unrelated donor is not matched at one of the six major transplantation antigens, the incidence of graft rejection and/or fatal GvHD increases to 60%.

GvHD is a disease with significant morbidity. Patients who develop acute GvHD may develop blisters covering most of their skin surface, massive gastrointestinal bleeding or fulminant liver failure and jaundice. Patients who develop chronic GvHD may develop scleroderma that results in joint contractures and skin ulcers, hair loss and a generalized wasting syndrome. Patients with acute or chronic GvHD are immuno-suppressed and at risk for life-threatening opportunistic infections similar to those that develop among AIDS patients.

Currently, existing transplantation technology involves the depletion of T cells, based on studies showing that the removal of such cells results in a decreased incidence of graft vs. host reactions. The T cells in the donor bone marrow are removed using one of several available techniques, such as removal of CD3$^+$ cells, E rosetting, and the use of soybean agglutinin. Alternatively, a clinical trial on allogeneic transplants has been conducted using enrichment of the donor bone marrow by a positive selection for hematopoietic stem cells based on the presence of the surface CD34 marker, by which many T cells are removed. In this trial, the CD34$^+$ selection was followed by removal of CD2$^+$ cells, which depleted an additional population of T and natural killer (NK) cells (Clarke, E., Potter, M.N., Steward, C. G., Cornish, J.M., Marks, D., Oakhill, A., and Pamphilon, D.H. 1997 *Blood* 90(10)Suppl. 1; page 37A; Abst. No. 1662; "Double T-cell depletion using sequential +/immunoaffinity or CD34+ cell selection followed by campath-M: Effect on CD34+ and progenitor cell recovery"; Bunjes, D., M.Wiesneth, S. Von Harsdorf, J. Duyster, T. Shreiner, C. Duncker, B. Macari, B. Kubanek 1997 1997 *Blood* 90(10)Suppl. 1; page 1034; Abst. No. 454; "T cell depleted allogeneic PBSC transplants in 30 patients with hematological malignancies: Benefits and Risks"). With current CD34$^+$ selection devices, there is an average of 2-3 logs T-cell depletion; e.g. a typical transplant of a CD34$^+$-selected graft can contain 2-4 x 10$^6$ CD34$^+$ cells and 0.1-0.5 x 10$^6$ CD3$^+$ (i.e. T - cells) per kg body weight recipient. Even with this treatment, however, such patients require GvHD prophylaxis.

Additionally, while removal of T cells from the bone marrow transplanted from matched unrelated or unmatched sibling donors reduces GvHD, it is also correlated with an increased incidence of rejection of the allogeneic bone marrow graft by the patient. Thus, lymphocytes, and especially T cells, present in the allogeneic bone marrow graft are important to ensure engraftment in antigenically and genetically mis-matched recipients. T cells present in the allogeneic graft also have an important role in eliminating residual cancer cells in the recipient, a phenomenon termed "graft vs. leukemia effect."

To minimize the disadvantageous effects of administering T cells, Waller et al. administered nonproliferating T cells that enhance engraftment in the absence of a GvHD response (U.S. Patent No. 5,800,539). In another clinical study, Aversa et al. (*New Engl. J. Med*. 339: 1186-1193) administered additional doses of donor peripheral blood mononuclear cells that had been T-cell depleted (using E-rosetting) and subsequently CD34$^+$ selected to recipients receiving a full-haplotype mismatch donor graft consisting of bone marrow or peripheral blood mononuclear cells.

Thus, while transplantation techniques have steadily improved since the first successful bone marrow transplantation in humans was conducted 30 years ago, patients receiving allografts still die from graft rejection and graft versus host disease, in addition to other complications. There remains a compelling need to develop an optimal procedure for allogeneic bone marrow transplantation.

The present invention is based on the discovery of a family of related cell types in the donor graft whose presence is correlated with decreased graft survival. Removal of one or more of these cell types, which are referred to as dendritic cells and dendritic cell progenitors or precursors (DCP), increases survival of allogeneic transplants in recipients.

**SUMMARY OF THE INVENTION**

The present invention provides the use, for the manufacture of a medicament for reducing or preventing graft versus host disease in an allogeneic hematopoietic system reconstituting cells transplant recipient, of allogeneic hematopoietic system reconstituting cells in which the number of dendritic cells has been effectively reduced by removing CD3 - (negative) cells having one or more of the cell surface markers CD123, CD45RA, CD4 and CD36 without removing CD3$^+$ (positive) cells, thereby reducing or preventing graft versus host disease.

Also described is a method of increasing the chance of survival for an allogeneic hematopoietic system reconstituting cells transplant recipient comprising administering to the recipient allogeneic hematopoietic system reconstituting cells in which the number of dendritic cells has been effectively reduced, thereby improving the chance for survival of the recipient. The invention additional describes a method for increasing the chance of survival for an allogeneic bone marrow transplant recipient comprising administering to the recipient allogeneic hematopoietic system reconstituting cells in which the number of dendritic progenitor cells administered is less than 0.25 x 10$^6$/kg body weight of the recipient. Also described is a column system for preparing hematopoietic system reconstituting cells prior to their transplantation comprising a first column containing a support to which are bound antibodies which specifically bind CD34 and a second column, containing a support to which are bound antibodies which specifically bind cell surface markers expressed on dendritic cell progenitors, wherein the first column adsorbs at least 30% of the CD34$^+$ cells as the hematopoietic system reconstituting cells are passed over the first column, and the adsorbed cell fraction released from the first column is passed over the second column and cells expressing surface markers for dendritic cell progenitors are adsorbed, such that the number of dendritic progenitor cells is effectively reduced.

**DETAILED DESCRIPTION OF THE INVENTION**

The present invention may be understood more readily by reference to the following definitions, detailed description of specific embodiments, and Examples included herein.

**DEFINITIONS**

"A" or "an" as used herein can mean one or more than one, depending upon the context in which it is used. For example, "a" cell can mean one cell or more than one cell.

"Dendritic cell" or "DC", as used herein, refers to a mature antigen presenting cell, which is identified by the expression of one or more of the following markers on its cell surface: CD1a, CD1b, and CD1c, CD4, CD11c, CD33, CD40, CD80, CD86, CD83 and HLA-DR, or a dendritic cell progenitor, as defined below, or both.

"Dendritic cell progenitor", "dendritic progenitor cells", "dendritic precursor cell", "dendritic cell precursor", or "DCP", used interchangeably herein, means a hematopoietic cell identified by the expression of one or more of the following markers on its cell surface: CD123, CD45RA, CD36, and CD4.

"Effectively reduced" means that the number of DC and/or DCP in a hematopoietic system reconstituting cells allograft is reduced such that the following equation is satisfied:

$$[\text{number of CD34}^+ \text{ cells]} - 2 \times [\text{number of DC/DCP}] \geq 1.5 \times 10^6 \text{ cells/kg body wt recipient.}$$

"Hematopoietic system reconstituting cells" means a population of cells, preferably human, that possess the capability of dividing and producing progeny that include all of the formed cellular elements of the blood. Sources of hematopoietic system reconstituting cells can include bone marrow (both fetal and adult) and peripheral blood mononuclear cells (PBMC).

"Donor" or "donor source" means the animal, preferably human, that is the natural source from which the hematopoietic system reconstituting cells are originally removed.

"Recipient" means the animal, typically human, into which the hematopoietic system reconstituting cells are transplanted..

"Allogeneic" means that the recipient is not the natural source from which the hematopoietic system reconstituting cells have been removed.

"Transplant", "allograft" and "graft" are used interchangeably herein and refer to the hematopoietic system reconstituting cells which are transplanted into a recipient.

## DETAILED DESCRIPTION

The present invention provides the use, for the manufacture of a medicament for increasing the chance for survival of an allogeneic hematopoietic system reconstituting cells transplant recipient of allogeneic hematopoietic system reconstituting cells in which the number of dendritic cells is effectively reduced by removing CD3- (negative) cells having one or more of the cell surface markers CD123, CD45RA, CD4 and CD36 without removing CD3$^+$ (positive) cells. That is, the ratio of the number of CD34$^+$ cells to the number of DC in the graft is in accordance with the following equation:

$$[\text{number of CD34}^+ \text{ cells}] - 2 \times [\text{number of DC}] \geq 1.5 \times 10^6 \text{ cells/kg body wt recipient.}$$

In one embodiment, the method of increasing the chance for survival of an allogeneic hematopoietic system reconstituting cells transplant recipient comprises administering to the recipient allogeneic hematopoietic system reconstituting cells in which the number of dendritic cell progenitors, or DCP, has been effectively reduced. That is, the ratio of the number of CD34$^+$ cells to the number of DCP in the graft is in accordance with the following equation:

$$[\text{number of CD34}^+ \text{ cells}] - 2 \times [\text{number of DCP}] \geq 1.5 \times 10^6 \text{ cells/kg body wt recipient.}$$

Representative combinations of CD34$^+$ cells and DCP reflecting an effectively reduced transplant are presented in Table 1.

Table 1.

| Number of cells in a graft of the invention. | | |
|---|---|---|
| No. of CD34$^+$ cells* | No. of DCPs* | df value* |
| 4.73 | 0.02 | 4.69 |
| 4.4 | 1.4 | 1.6 |
| 2.7 | 0.5 | 1.6 |
| 1.9 | 0.15 | 1.6 |
| 2.38 | 0.3 | 1.77 |
| 3.34 | 0.04 | 3.27 |

*x 10$^6$/per kg body weight recipient (df= difference function)

While not intending to be limited by theory, it is believed that the removal of dendritic cells from an allograft can reduce and perhaps prevent graft versus host disease (GvHD). Thus, in another embodiment, the present invention describes a method of reducing or preventing GvHD in an allogeneic hematopoietic system reconstituting cells transplant recipient comprising administering to the recipient allogeneic hematopoietic system reconstituting cells in which the number of dendritic cells has been effectively reduced. In another embodiment, reducing or preventing GvHD in an allogeneic hematopoietic system reconstituting cells transplant recipient comprises administering to the recipient allogeneic hematopoietic system reconstituting cells in which the number of dendritic cell progenitors (DCP) has been effectively reduced. In a specific embodiment, the present invention describes a method of reducing or preventing graft versus host disease in allogeneic bone marrow transplant recipients comprising administering to the patient allogeneic hematopoietic system reconstituting cells in which the number of dendritic progenitor cells administered is less than 0.25 x 10$^6$/kg body weight of the recipient.

In one embodiment, the hematopoietic system reconstituting cells to be transplanted are positively selected for CD34$^+$ phenotype, i.e. the expression of the CD34 cell surface marker. "Positively selected" means that cells expressing the CD34 protein are collected by methods well known in the art, such as fluorescence-activated cell sorting or immunomagnetic chromatography, and as described in the Examples herein and in the references herein cited. The positive selection for CD34$^+$ hematopoietic system reconstituting cells can be done prior to or after the depletion or removal of dendritic cells from hematopoietic system reconstituting cells.

The methods of this invention may be used in combination with other methods and processes for transplanting hematopoietic system reconstituting cells. For example, the methods herein disclosed may be used in combination with the method of Waller et al. (U.S. Patent No. 5,800539) which comprises administering nonproliferating T cells that enhance engraftment to improve allogeneic hematopoietic cell transplantation. Alternatively, or in further combination with the Waller et al. method comprising administering nonproliferating T cells, the methods described herein may be used in combination with methods described by Aversa et al. (*New Engl. J. Med.* 339: 1186-1193) comprising administering additional doses of T-cell depleted, CD34$^+$ donor peripheral blood mononuclear cells to improve allogeneic hematopoietic cell transplantation.

The efficacy of this invention can be demonstrated by the increased number of survivors, surveyed, for example, at two or three years post-transplant, in the group of recipients of transplanted hematopoietic system reconstituting cells according to the methods of this invention, as compared to groups of recipients of transplanted hematopoietic system reconstituting cells not in accordance with these methods. The efficacy of this invention in reducing or preventing graft versus host disease can be determined in a clinical setting by measuring a number of clinical indices as would be well known to the clinician, such as time to full engraftment, or decreased incidence of graft failure.

## Markers for Dendritic Cells and Dendritic Progenitor Cells

Dendritic cells (DC) are antigen presenting cells which are important in the initiation of immune responses. Based on *in vitro* culturing and characterization, DC are thought to represent a family of related cell types, perhaps from a single lineage. Progenitors of DC (DCP) can now be identified in many tissues, such as bone marrow and blood, based on the expression of certain cell surface markers. One type of precursor cell, the Langerhans cell, is found in the epidermis. Work on Langerhans cells established that these immature precursors are capable of antigen uptake, but do not demonstrate a significant ability to stimulate T cells until they differentiate into mature DC.

A variety of markers have been identified that are useful in defining certain cell types of the dendritic cell family. For example, markers expressed on the surface of mature dendritic cells can include CD1a, CD11c, DEC-205, CD4, CD33, CD40, CD80, CD83, and CD86. Typically, the following markers are not found on the surface of mature dendritic cells: CD20, CD3, CD14, CD16, CD2, CD45RA, CD56, and CD19. Cell surface markers present on dendritic cell precursors include CD4, CD45RA, CD34, CD10, CD123, and CD36, while the following markers are not found on the surface of dendritic cell precursors: CD 11c, CD1a, CD8, CD40, CD80, CD83 and CD86 (see for example Caux et al. *Intl. Immunology* 1994 6(8): 1177-1185; Steptoe et al. *J. Immunology* 1997 159:5483-5491; Grouard et al. (*J. Exp. Med.*1997 185(6):1101-1111; O'Doherty et al. *Immunology* 1994 82: 487-493; Galy et al., *J. Immunotherapy* 1998 21 (2):132-141; Strunk et al., J. Exp. Med. 1997 185(6): 1131-1136; Olweus et al. *Proc. Natl. Acad. Sci. USA* 1997 94: 12551-12556; Prignano et al., *Intl. J. Dermatol.* 1998 37:116-119). By the appropriate use of these markers, dendritic cells can be distinguished from dendritic cell progenitors.

## Methods for Removing and/or Reducing the Number of DC and/or DCP in Hematopoietic System Reconstituting Cells

The removal of dendritic cells and/or dendritic cell precursors from hematopoietic system reconstituting cells can be accomplished by a number of protocols, using cell sorting and collection techniques that are well known in the art (e.g. Olweus, J. et al. 1997 *Proc. Natl. Acad. Sci. USA* 94:12551-12556), including but not limited to fluorescence-activated cell sorting (FACS) and immunomagnetic chromatography.

For example, to remove DC from hematopoietic system reconstituting cells, the hematopoietic system reconstituting cells are first contacted with fluorescently labeled, e.g. FITC-labeled, antibodies that specifically bind to one of the mature dendritic cell markers, i.e. CD1a, CD11c, DEC-205, CD4, CD33, CD40, CD80, CD83, and CD86, and are then subjected to fluorescence-activated flow cytometry. Cells which show an FITC signal above the second decade, on a four decade logarithmic plot, are removed, while cells which are not bright, including CD34+ cells, are collected for use in a transplantation.

An overall goal for the protocols employed for the removal of DCP is to remove the population of cells that have a CD3$^-$, CD4$^+$, CD8$^-$ phenotype, which is further characterized by having a lower side-scatter in a FACS (fluorescence-activated cell sorting) plot than the typical monocyte population. However, this population can be identified and removed based on a number of different phenotypic markers, as detailed below.

One protocol for effectively reducing the number of DCP is to remove CD123$^+$ cells, the CD123 surface protein being the alpha chain for interleukin 3 (IL-3) receptor (IL-3R-$\alpha$). As an example, antibodies which specifically bind CD123 (available from PharMingen/Becton-Dickinson, San Diego, CA), attached to paramagnetic beads, can be contacted with hematopoietic system reconstituting cells, in a bead to target cell ratio of approximately 50:1, followed by bead removal from the hematopoietic system reconstituting cells using a fixed magnet or magnetized material. In another embodiment, antibodies which specifically bind CD123 are bound to an immobilized support, and the hematopoietic system reconstituting cells are passed over the support, with the eluent cells being DCP-depleted. In another embodiment, DCP can be removed by fluorescence-activated cell sorting following binding of the IL-3R-$\alpha$/CD123 protein with a fluorescently labeled monoclonal antibody. In these embodiments, the resulting hematopoietic system reconstituting cells to be used in transplantation fail to express CD123 but include CD34$^+$ cells.

DCP can also be removed from hematopoietic system reconstituting cells on the basis of the presence of the CD4 protein on the cell surface. In a specific embodiment, a fluorescently labeled, anti-CD4 antibody is added to hematopoietic system reconstituting cells. The hematopoietic system reconstituting cells are subjected to fluorescence-activated flow cytometry, and the cells in which the CD4 signal is above the second decade, on a four decade logarithmic plot, are removed, while cells which fail to express CD4, including CD34$^+$ cells, are collected.

Similarly, other ligands useful in removing dendritic cells and/or dendritic cell progenitors can be attached to paramagnetic beads which are contacted with hematopoietic system reconstituting cells, which beads are then removed via a magnet. Ligands can be any molecule that binds cell surface proteins. For example, a ligand can be an antibody, a cytokine, or a hormone. Ligand antibodies can be polyclonal or monoclonal and can be produced by methods well-known in the art.

Alternatively, the ligand can be bound to a support over which the hematopoietic system reconstituting cells are passed and unbound cells collected. In another embodiment, the ligand can be conjugated with a fluorescent label, allowing the removal of labeled cells from the hematopoietic system reconstituting cells via sorting through fluorescence-activated cytometry. The result of all of these protocols is DC/DCP-depleted hematopoietic system reconstituting cells. In addition to anti-CD123 or anti-CD4, other ligand(s) useful in removing DCPs in the present invention are CD45RA antibodies and CD36 antibodies. Any of the ligands of this invention can be used in combination or alone, to obtain hematopoietic system reconstituting cells in which the dendritic cell population has been effectively reduced.

Alternatively, IL-3, as a ligand for the CD123 surface protein, can itself be used to remove DCP from hematopoietic system reconstituting cells. IL-3 can be bound to a column or attached to paramagnetic beads, as described above for the antibody ligands, to remove the DCP from the graft. In another embodiment, IL-3 is conjugated to a toxic moiety and incubated with the hematopoietic system reconstituting cells. The IL3-toxic moiety conjugate binds to IL3R-$\alpha$ on the surface of DCP and is taken into the cell, which is killed by the toxic moiety. Thus, DCP are eliminated from hematopoietic system reconstituting cells by selective cell killing. Any moiety which is toxic to human cells and which can be bound covalently to IL-3 can be used; examples of toxic moieties include but are not limited to, diptheria toxin, ricin, and pertussis toxin, as well as any other toxins known in the art.

Any one or a combination of these techniques for removing DC/DCP can be employed so that the number of DC/DCP in an allograft of hematopoietic system reconstituting cells is effectively reduced. For example, an allograft can be treated, by one or more of the techniques described above, to remove CD4$^+$ and CD123$^+$ cells. Alternatively, an allograft can be treated to remove any one of the following combinations: CD123$^+$ and CD45RA$^+$ cells; CD123$^+$ and CD36$^+$ cells; CD45RA$^+$ and CD36$^+$ cells; CD123$^+$, CD4$^+$ and CD45RA$^+$ cells; CD123$^+$, CD4$^+$, and CD36$^+$; or CD4$^+$, CD123$^+$, CD45RA$^+$ and CD36$^+$ cells. In a specific embodiment, the allograft is initially positively selected for cells that express the CD34 marker on their surface, and such CD34 positively selected cells are subsequently treated to remove cells expressing cell surface proteins in any one of the following combinations: CD123$^+$ and CD45RA$^+$ cells; CD4$^+$ and CD123$^+$ cells; CD123$^+$ and CD36$^+$ cells; CD45RA$^+$ and CD36$^+$ cells; CD123$^+$, CD4$^+$ and CD45RA$^+$ cells; CD123$^+$, CD4$^+$, and CD36$^+$; or CD4$^+$, CD123$^+$, CD45RA$^+$ and CD36$^+$ cells.

Using any of the foregoing techniques for DCP removal, the present invention provides a composition suitable for transplanting into a recipient in need of a transplant of hematopoietic system reconstituting cells comprising hematopoietic system reconstituting cells in which the number of dendritic cell progenitors has been effectively reduced.

Also described in this invention are columns and column systems for preparing hematopoietic system reconstituting cells prior to their transplantation such that the number of DCP in the transplant is effectively reduced. As an example, a dual column system is described comprising a first column containing a support to which are bound antibodies which specifically bind CD34 and a second column containing a support to which are bound antibodies which specifically bind cell surface markers expressed on dendritic cell progenitors, wherein the first column adsorbs at least thirty percent (30%) of the CD34$^+$ cells. The adsorbed CD34$^+$ cells, released from the first column (e.g. by agitation) are then passed over the second column, which contains sufficient bound antibodies to adsorb cells expressing surface markers for DCP, so that the number of DCP is effectively reduced in the eluent (pass-through) fraction of the second column. The cell content of a transplant after treatment by this dual column system contains at least 2 x 10$^6$ CD34$^+$ cells/kg and less than 0.25 x 10$^6$ DCP/kg body weight of the recipient.

Another column for preparing hematopoietic system reconstituting cells prior to their transplantation such that the number of DCP is effectively reduced, comprises a support to which is bound antibodies which specifically bind to a combination of cell surface markers selected from the group of combinations consisting of: CD4 and CD123; CD123 and CD45RA; CD 123 and CD36; CD45RA and CD36; CD123, CD4 and CD45RA; CD123, CD4, and CD36; or CD4, CD123, CD45RA and CD36. The column contains sufficient antibodies against cell surface markers on DCPs so that the number of DCPs in the hematopoietic system reconstituting cells is less than 0.25 x 10$^6$ DCPs/kg body weight of the recipient.

The following examples are intended to illustrate, but not limit, the invention. While they are typical of those that might be used, other procedures known to those skilled in the art may be alternatively employed.

**EXAMPLES**

**Example 1. <u>Transplant Patient Survey</u>**

A statistical survey of the transplant, or allograft, characteristics of 104 patients who received non-T-cell depleted allogeneic bone marrow transplants from HLA-matched sibling donors was conducted. The pre-transplant conditioning of these patients included: busulfan-based treatments (n=89), total body irradiation with cyclophosphamide (n=10), and cyclophosphamide (n=5). The total number of cells in the grafts averaged 3 x $10^8$ cells/kg body weight recipient. The number of CD34$^+$ cells was correlated with improved survival (p=0.0004 for overall survival and p=0.001 for disease-free survival). In addition, a population of cells in the transplants, characterized as being CD3$^-$, CD4$^{+(hi)}$, CD8$^-$, low side scatter, referred to herein as DCPs, was quantified, and the number of such cells in the transplants was negatively correlated with overall survival (OS) and disease free survival (DFS, p = 0.005). In this survey,donor age and gender, CFU-GM (ranging from 0-41 x $10^4$ per kg body weight), total CFU (ranging from 4-282 x $10^4$ per kg), and the relative numbers of T-cell subsets, NK cells, B-cells and monocytes were not significant variables in any multivariate models.

Thus, a simple mathematical relationship (a difference function, df) can be described, based on the number of CD34$^+$ cells and the number of DCPs in the graft, as follows: df = (number of CD34$^+$ cells) - 2x(number of DCP). A threshold value of 1.5 x $10^6$ per kg body wt recipient for df separated these patients into two groups: those patients whose grafts have df greater than or equal to 1.5 x $10^6$ per kg body wt recipient had a 67% DFS at 3 years, compared to 34% DFS for patients with grafts in which the df was less than 1.5 x $10^6$ per kg body wt recipient.

**Example 2. <u>Transplantation of Selected Stem Cells</u>**

<u>Donor Source</u>: Bone marrow or peripheral blood stem cells (PBSC) collected from an HLA matched or a non-HLA matched donor.

<u>Donor PBSC Collection</u>: To mobilize PBSC, G-CSF is administered daily until leukapheresis is completed. Donors receive G-CSF 10 ug/kg/day subcutaneously, based on the donor's actual body weight. During mobilization and leukapheresis, institutional standards for donor supportive care should be maintained. PBSC are collected through leukapheresis. Leukapheresis will be performed on a continuous flow separator machine per institution standard method. The endpoint of each leukapheresis collection will be either the processing of a minimum of 12 liters whole blood or 4 hours of leukapheresis. Leukapheresis will begin on Day $^+$ 5 of donor G-CSF therapy and will be performed daily (maximum of four days of leukapheresis) until the target of 2 x $10^6$ CD34$^+$ cell/kg recipient's actual weight is obtained (after processing). The CD34$^+$ cell number for determining whether to continue apheresis, will be based on flow cytometry assays done daily.

<u>Donor Cell Preparation</u>: Bone marrow or PBSC are selected using an immunomagnetic adsorption cell separation system, such as the Isolex 300 system (Baxter Immunotherapy, Irvine, CA) following published protocols (e.g. Kvalheim G, Pharo A, Holte H et al. The use of immunomagnetic beads and Isolex 300 gives high purity and yield of CD34$^+$ cells from peripheral blood progenitor cell products. Bone Marrow Transplant 1996;17 (suppl 1):S57; and Cancelas JA, Canais C, Querol S et al. CD34$^+$ cell transplantation from mobilized peripheral blood by immunomagnetic adsorption in breast cancer patients. Bone Marrow Transplant 1996;17 suppl 1):S32) or the next-generation Isolex 300i system, which is a fully automated operation, following published protocols (Report Series 8, Initial Performance of Automated Isolex 300i System in Multicenter International clinical Trials. I. Purity). These devices result in a final graft in which the mean purity ranges between 77-93% with a post-selection cell count of 1.1-6.4 x $10^6$ CD34$^+$ cells per kg. Additional aliquots of the graft are cryopreserved in a vapor phase of liquid nitrogen for further infusions, if appropriate.

The CD34$^+$ selected graft is subsequently treated to remove DCP (see Example 3 below). The resulting CD34$^+$, DCP-depleted graft, containing at least 2 x$10^6$ CD34$^+$ cells/kg recipient body and less than 0.25 x $10^6$ DCP per kg, is infused into the recipient. Additional aliquots of the graft are cryopreserved in a vapor phase of liquid nitrogen for further infusions, if appropriate.

<u>Donor Cell Transplantation</u>: Donor cells, prepared as above, are administered to the patient in need of such a transplant, through a central venous catheter (typically in the chest). Patients receiving these donor cells may undergo a preconditioning treatment one to two days prior to infusion of the donor cells. Such preconditioning treatments would be either total body irradiation or busulfan in combination with immunosuppressive therapies such as fludarabine, thiotepa, cyclophosphamide and antibodies to deplete T cells.

**Example 3. <u>Cell Sorting</u>**

**General protocol for cell sorting**. Cells were stained using FITC-conjugated CD34-specific monoclonal antibodies and PE-conjugated monoclonal antibodies recognizing CD4, CD123, CD36, or CD45RA (Becton-Dickinson, San Jose, CA). Directly-conjugated isotype-matched irrelevant antibodies served as controls. Cells were stained for 30 min at 4 °C in staining buffer consisting of Hank's buffered saline solution (HBSS, Mediatech, Hemdon, VA) containing 2% v/v autologous human plasma or 1% human serum albumin. Excess antibodies were removed by dilution with 10 volumes of staining buffer, followed by centrifugation at 500x g. Stained cells were immediately analyzed using a FACSCAN flow cytometer (BDIS, San Jose, CA), or sterile-sorted using a FACSVANTAGE cell sorter (BDIS, San Jose, CA). List-mode data were acquired using forward- and side-scatter gates appropriate for viable nucleated cells. Data analysis was performed using CellQuest™ or LYSIS-II software (BDIS, San Jose, CA). CD34+ DCP- cells were sorted by setting the orthogonal sort gate to include cells expressing CD34 (an FITC-positive event) and to exclude cells expressing a DCP marker (a PE-positive event), and collecting the CD34+ DCP- cells in a sterile tube..

**Example 4. <u>Characterization of isolated DCP</u>**

Dendritic cells were isolated and purified from bone marrow by magnetic column separation, using a Miltenyi Biotec MACS system to absorb (deplete) lineage positive cells expressing lymphoid, granulocyte, or monocyte markers (i.e. Lineage⁻). Subsequent cell sorting of the Lineage⁻ population using CD123-PE and CD11c-APC (FACS Vantage System, with a turbo sort option, using a primary Enterprise 488 argon laser and secondary dye laser) showed two distinct populations of dendritic cells based on the expression pattern of these two antigens on the cell surface: DC1, which were CD11c⁺ and CD123⁻, and DCP2, which were CD123⁺ and CD11c⁻. Further analysis of these two subtypes showed that the DCP2 population failed to express the co-stimulatory molecules CD80, CD86 and CD40, while the DC1 population showed expression of these markers. As discussed herein, these three markers are known to be expressed on mature dendritic cells, but not on dendritic cell precursor cells. A scatter plot showing the correlation between two analysis methods disclosed herein for dendritic cell precursors, i.e. CD4⁺/CD3⁻, low side scatter as one method, and CD 11c⁻/CD123⁺ as the second method, showed an excellent correlation (coefficient = 0.82) confirming that these markers identify the same cell population.

**Claims**

1. Use of allogeneic hematopoietic system reconstituting cells in which the number of dendritic cells has been effectively reduced for the manufacture of a medicament <u>for increasing the chance of survival</u> in a patient in need of an allogeneic hematopoietic system reconstituting cell transplant, wherein the number of dendritic cells has been effectively reduced by removing CD3- (negative) cells having one or more of the cell surface markers CD123, CD45RA, CD4 and CD36 without removing CD3⁺ (positive) cells.

2. The use of claim 1, wherein the dendritic cells are dendritic cell progenitors.

3. The use of claim 2, in which the number of dendritic cell progenitors administered is less than 0.25 x 10⁶/kg body weight of the recipient.

4. The use of claim 1, wherein the dendritic cells are removed using fluorescence-activated cell sorting.

5. The use of claim 1, wherein the dendritic cells are removed by a) contacting the hematopoietic system reconstituting cells with a ligand which specifically binds a receptor on the surface of dendritic cells, the ligand being bound to a paramagnetic support, and b) removing the dendritic cells from the hematopoietic system reconstituting cells with a magnet.

6. The use of claim 1, wherein the dendritic cells are removed by passing the hematopoietic system reconstituting cells through a column or columns containing support material to which are bound one or more ligands which bind to one or more proteins expressed on the surface of a dendritic cell.

7. The use of claim 5, wherein the ligand is selected from the group consisting of IL3, anti-CD123, anti-CD45RA, anti-CD4, and anti-CD36.

8. The use of claim 6, wherein the ligand is selected from the group consisting of IL3, anti-CD123, anti-CD45RA, anti-CD4, and anti-CD36.

9. The use of claim 2, wherein the dendritic cell progenitors are removed by contacting the hematopoietic system reconstituting cells with an IL-3-toxic moiety conjugate, whereby the dendritic cell progenitors are destroyed by the toxic moiety.

10. The use of claim 9 wherein the toxic moiety is selected from the group consisting of diphtheria toxin, ricin, and pertussis toxin

**Patentansprüche**

1. Verwendung von das allogene hematopoietische System rekonstituierenden Zellen, bei denen die Anzahl dentritischer Zellen wirksam reduziert worden ist, zur Herstellung eines Medikaments zur Erhöhung der Überlebenswahrscheinlichkeit bei einem Patienten, der eine das allogene hematopoietische System rekonstruierende Zelltransplantation benötigt, wobei die Anzahl dentritischer Zellen wirksam reduziert worden ist durch Entfernen von CD3$^-$ (negativen)-Zellen, die einen oder mehrere der Zelloberflächenmarker CD123, CD45RA, CD4 und CD36 aufweisen, ohne CD3$^+$ (positive)-Zellen zu entfernen.

2. Verwendung von Anspruch 1, wobei die dendritischen Zellen Vorläufer dendritischer Zellen sind.

3. Verwendung von Anspruch 2, wobei die Zahl der verabreichten Vorläufer dendritischer Zellen weniger als $0,25 \times 10^6$/ kg Körpergewicht des Empfängers beträgt.

4. Verwendung von Anspruch 1, wobei die dendritischen Zellen unter Verwendung der Fluoreszenz-aktivierten Zell-Sortierung entfernt sind.

5. Verwendung von Anspruch 1, wobei die dendritischen Zellen entfernt sind durch a) Kontaktieren der das hematopoietische System rekonstituierenden Zellen mit einem Liganden, der spezifisch einen Rezeptor auf der Oberfläche von dendritischen Zellen bindet, wobei der Ligand an einen paramagnetischen Träger gebunden ist, und b) Entfernen der dendritischen Zellen von den das hematopoietische System rekonstituierenden Zellen mit einem Magneten.

6. Verwendung von Anspruch 1, wobei die dendritischen Zellen entfernt sind durch Passieren der das hematopoietische System rekonstituierenden Zellen durch eine Säule oder Säulen, die ein Trägermaterial enthält (enthalten), an welches ein oder mehrere Ligand(en) gebunden ist (sind), der (die) ein oder mehrere auf der Oberfläche einer dendritischen Zelle exprimiertes (exprimierte) Protein(e) bindet (binden).

7. Verwendung von Anspruch 5, wobei der Ligand aus der aus IL3, anti-CD123, anti-CD45RA, anti-CD4 und anti-CD36 bestehenden Gruppe ausgewählt ist.

8. Verwendung von Anspruch 6, wobei der Ligand aus der aus IL3, anti-CD123, anti-CD45RA, anti-CD4 und anti-CD36 bestehenden Gruppe ausgewählt ist.

9. Verwendung von Anspruch 2, wobei die Vorläufer der dendritischen Zellen entfernt sind durch Kontaktieren der das hematopoetische System rekonstituierenden Zellen mit einem Konjugat von IL-3 und einem toxischen Strukturelement, wobei die Vorläufer der dendritischen Zellen durch das toxische Strukturteil zerstört werden.

10. Verwendung von Anspruch 9, wobei das toxische Strukturelement aus der aus Diphterie-Toxin, Rizin und Pertussis-Toxin bestehenden Gruppe ausgewählt ist.

**Revendications**

1. Utilisation de cellules allogéniques de reconstitution du système hématopoïétique dans lesquelles le nombre de cellules dentritiques a été efficacement réduit en vue de la fabrication d'un médicament visant à augmenter les chances de survie d'un patient receveur de greffe de cellules allogéniques de reconstitution du système hématopoïétique, dans laquelle le nombre de cellules dentritiques a été efficacement réduit par la séparation des cellules CD3$^-$ (négatives) portant un ou plusieurs des marqueurs antigéniques CD123, CD45RA, CD4 et CD36 sans séparation des cellules CD3$^+$ (positives).

2. Utilisation selon la revendication 1, dans laquelle les cellules dentritiques sont des cellules souches de cellules dentritiques.

3.  Utilisation selon la revendication 2, dans laquelle le nombre de cellules souches de cellules dentritiques administrées est inférieur à 0,25 X $10^5$/kg de poids corporel du receveur.

4.  Utilisation selon la revendication 1, dans laquelle les cellules dentritiques sont séparées en utilisant un trieur de cellules à fluorescence.

5.  Utilisation selon la revendication 1, dans laquelle les cellules dentritiques sont séparées par a) la mise en contact des cellules de reconstitution du système hématopoïétique avec un ligand qui se lie spécifiquement à un récepteur sur la surface des cellules dentritiques, le ligand étant lié à un support paramagnétique, et b) la séparation des cellules dentritiques des cellules de reconstitution du système hématopoïétique à l'aide d'un aimant.

6.  Utilisation selon la revendication 1, dans laquelle les cellules dentritiques sont séparées par le passage des cellules de reconstitution du système hématopoïétique à travers une colonne ou des colonnes contenant un support auquel sont liés un ou plusieurs ligands qui se lient à une ou plusieurs protéines exprimées sur la surface d'une cellule dentritique.

7.  Utilisation selon la revendication 5, dans laquelle le ligand est choisi dans le groupe constitué de IL-3, anti-CD123, anti-CD45RA, anti-CD4 et anti-CD36.

8.  Utilisation selon la revendication 6, dans laquelle le ligand est choisi dans le groupe constitué de IL-3, anti-CD123, anti-CD45RA, anti-CD4 et anti-CD36.

9.  Utilisation selon la revendication 2, dans laquelle les cellules souches de cellules dentritiques sont séparées par le contact des cellules de reconstitution du système hématopoïétique avec un conjugué IL-3 - fragment toxique, moyennant quoi les cellules souches de cellules dentritiques sont détruites par le fragment toxique.

10. Utilisation selon la revendication 9, dans laquelle le fragment toxique est choisi dans le groupe constitué de toxine de la diphtérie, ricine et toxine de la coqueluche.